# EUROPÄISCHE PATENTSCHRIFT

(11) **EP 0 835 863 B1**
(45) Veröffentlichungstag und Bekanntmachung des Hinweises auf die Patenterteilung: **18.12.2002**
(21) Anmeldenummer: 97117216.8
(22) Anmeldetag: 04.10.1997
(51) Int. Cl.: C07C 219/06, C11D 1/62, A61K 7/06

(54) **Esterquats**
Esterquats
Esterquats

(30) Priorität: 11.10.1996 DE 19642038
(43) Veröffentlichungstag der Anmeldung: 15.04.1998
(73) Patentinhaber: Cognis Deutschland GmbH & Co. KG, 40589 Düsseldorf (DE)
(72) Erfinder: Llosas Bigorra, Joaquim, 08203 Sabadell (ES); Pi Subirana, Rafael, 08400 Granollers (ES); Bonastre Gilabert, Nuria, 08210 Barberà del Vallés (ES)

(56) Entgegenhaltungen:
- EP-A- 0 718 275
- WO-A-91/01295
- DE-A- 4 111 966

## Beschreibung

### Gebiet der Erfindung

Die Erfindung betrifft neue Esterquats mit verbessertem rheologischen Verhalten auf Basis von definierten Alkanolaminmischungen, ein Verfahren zu ihrer Herstellung sowie deren Verwendung zur Herstellung von Avivagemitteln und kosmetischen Zubereitungen.

### Stand der Technik

Unter der Bezeichnung "Esterquats" werden im allgemeinen quaternierte Fettsäuretriethanolaminestersalze verstanden. Es handelt sich dabei um bekannte Stoffe, die man nach den einschlägigen Methoden der präparativen organischen Chemie erhalten kann. In diesem Zusammenhang sei auf die Internationale Patentanmeldung **WO 91/01295** (Henkel) verwiesen, nach der man Triethanolamin in Gegenwart von unterphosphoriger Säure mit Fettsäuren partiell verestert, Luft durchleitet und anschließend mit Dimethylsulfat oder Ethylenoxid quaterniert. Aus der Deutschen Patentschrift **DE-C1 43 08 794** (Henkel) ist überdies ein Verfahren zur Herstellung fester Esterquats bekannt, bei dem man die Quaternierung von Triethanolaminestem in Gegenwart von geeigneten Dispergatoren, vorzugsweise Fettalkoholen, durchführt. Übersichten zu diesem Thema sind beispielsweise von R.Puchta et al. in **Tens.Surf.Det., 30, 186 (1993),** M.Brock in **Tens.Surf.Det. 30, 394 (1993),** R.Lagerman et al. in **J.Am.Oil.Chem.Soc., 71, 97 (1994)** sowie I.Shapiro in **Cosm.Toil. 109, 77 (1994)** erschienen. Üblicherweise werden die Esterquats als alkoholische Konzentrate in den Handel gebracht und anschließend auf die Anwendungskonzentration verdünnt. Dabei besteht das Problem, daß die Lösungen für manche Anwendungen zu dickflüssig sind und Viskositätsregulatoren eingesetzt werden müssen.

Abgesehen davon, daß die Additivierung der Esterquats mit weiterem technischen Aufwand und Kosten verbunden ist, wird die Mitverwendung derartiger Additive nicht immer gewünscht, so daß im Markt ein hohes Interesse an Esterquats besteht, die in wäßriger Verdünnung ohne Mitverwendung von Viskositätsregulatoren eine ausreichend niedrige und auch bei Temperaturlagerung konstante Viskosität zeigen. Die Aufgabe der Erfindung hat darin bestanden, diesem Bedürfnis Rechnung zu tragen.

### Beschreibung der Erfindung

Gegenstand der sind Esterquats, die man erhält, indem man Mischungen von Methyldiethanolamin (MDA) und Triethanolamin (TEA) im Gewichtsverhältnis 20 : 80 bis 1 : 99 mit Fettsäuren verestert und die Reaktionsprodukte anschließend in an sich bekannter Weise mit Alkylierungsmitteln quatemiert.

Überraschenderweise wurde gefunden, daß die erfindungsgemäßen Esterquats auf Basis von MDA/TEA-Mischungen in wäßriger Verdünnung gegenüber bekannten Produkten auf Basis reinem Triethanolamins eine auch bei Lagerung konstant niedrige Viskosität zeigen, während sie gegenüber ebenfalls bekannten Produkten auf Basis reinen Methyldiethanolamins einen besseren Weichgriff und eine verbesserte biologische Abbaubarkeit zeigen. Die Erfindung schließt die Erkenntnis ein, daß der Eigenschaftsverlauf bei Esterquats auf Basis von MDA/TEA-Mischungen nicht, wie man hätte erwarten sollen, linear ist, sondern schon der Zusatz von sehr geringen Mengen MDA bei der Herstellung der Fettsäurealkanolaminestern auf Basis TEA zu einer synergistischen Abnahme der Viskosität führt.

Ein weiterer Gegenstand der Erfindung betrifft ein Verfahren zur Herstellung von Esterquats, bei dem man Mischungen von Methyldiethanolamin und Triethanolamin im Gewichtsverhältnis 20 : 80 bis 1 : 99 mit Fettsäuren der Formel (I) in der R¹CO für einen aliphatischen, gesättigten und/oder ungesättigten Acylrest mit 6 bis 22 Kohlenstoffatomen steht verestert und die Reaktionsprodukte anschließend in an sich bekannter Weise mit Alkylierungsmitteln quaterniert.

### Alkanolaminmischungen

Die im Sinne der Erfindung einzusetzenden Alkanolaminmischungen können technisches Methyldiethanolamin und technisches Triethanolamin im Gewichtsverhältnis 20 : 80 bis 1 : 99, vorzugsweise 15 : 85 bis 8 : 92 enthalten.

### Fettsäuren

Zur Herstellung der erfindungsgemäßen neuen Esterquats werden üblicherweise Fettsäuren eingesetzt, die der Formel **(I)** folgen,

**R**^{**1**}**COOH** **(I)**

in der R¹CO für einen aliphatischen, gesättigten und/oder ungesättigten Acylrest mit 6 bis 22 Kohlenstoffatomen steht. Typische Beispiele sind Capronsäure, Caprylsäure, 2-Ethylhexansäure, Caprinsäure, Laurinsäure, Isotridecansäure, Myristinsäure, Palmitinsäure, Palmoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen, die z.B. bei der Druckspaltung von natürlichen Fetten und Ölen oder bei der Reduktion von Aldehyden aus der Roelen'schen Oxosynthese anfallen. Vorzugsweise werden Fettsäuren mit 12 bis 18 Kohlenstoffatomen wie beispielsweise partiell gehärtete Palm- und/oder Talgfettsäuren eingesetzt, die eine Iodzahl im Bereich von 0,5 bis 50 aufweisen.

### Veresterung

Die Veresterung kann in an sich bekannter Weise, d.h. in Gegenwart von hypophosphoriger Säure oder deren Salzen und gegebenenfalls Reduktionsmittel wie etwa Natriumborhydrid durchgeführt werden. Im allgemeinen kann man die Alkanolamine und die Fettsäuren im Molverhältnis 1 : 1,1 bis 1 : 2,4 einsetzen, vorzugsweise liegt das Verhältnis im Bereich 1 : 1,4 bis 1 : 1,9. Die Reaktion wird üblicherweise bei Temperaturen im Bereich von 120 bis 180 °C durchgeführt. Falls gewünscht, kann die Reaktionsmischung anschließend durch Zugabe von Wasserstoffperoxid gebleicht werden.

### Alkylierung

Die Alkylierung der Fettsäurealkanolaminester kann in an sich bekannter Weise durchgeführt werden. Hierzu wird der Ester vorgelegt und mit dem Alkylierungsmittel - das man üblicherweise in äquimolaren Mengen oder leichtem Unterschuß einsetzt - bei erhöhten Temperaturen (40 bis 95 °C) gerührt. Die Reaktion kann in alkoholischer Lösung, beispielsweise in Isopropylalkohol, durchgeführt werden, es ist jedoch ebenso möglich, in Gegenwart inerter Emulgatoren bzw. Dispergatoren, beispielsweise Fettalkoholen oder Alkylpolyglucosiden und dergleichen, zu arbeiten. Die Auswahl richtet sich dabei nach der vorgesehenen Verwendung der Esterquats. Insbesondere für die Herstellung von Esterquats für den Einsatz in den Bereichen hair care und skin care ist das Arbeiten in Abwesenheit von niederen Alkoholen und die Mitverwendung von geeigneten Emulgatoren bzw. Dispergatoren deutlich bevorzugt. Nach Abschluß der Reaktion kann nichtumgesetztes Alkylierungsmittel durch Zugabe einer geringen Menge Aminosäure, vorzugsweise Glycin, zerstört werden. Als Alkylierungsmittel kommen in diesem Zusammenhang Alkylhalogenide, Dialkylsulfate und Ethylenoxid - letzteres in Gegenwart von Dialkylphosphaten - in Betracht. Vorzugsweise betrifft die Erfindung methylquaternierte Esterquats in Form ihrer Chloride und insbesondere Methylsulfat-Salze sowie Esterquat-Salze, die mit 1 bis 5 Mol Ethylenoxid quaterniert worden sind. Vorzugsweise führt man jedoch die Alkylierung mit Dimethylsulfat durch.

### Gewerbliche Anwendbarkeit

Die erfindungsgemäßen neuen Esterquats zeigen in wäßriger Verdünnung eine deutlich niedrigere Viskosität als vergleichbare bekannte Produkte auf Basis vom reinem Triethanolamin. Ein weiterer Gegenstand der Erfindung betrifft daher die Verwendung der neuen Esterquats zur Herstellung von Avivagemitteln für die Wäschevor- bzw. -nachbehandlung sowie von kosmetischen Zubereitungen, wie beispielsweise Haarshampoos, Haarspülungen, Cremes, Lotionen und dergleichen, in denen die Stoffe in Mengen von 1 bis 50, vorzugsweise 5 bis 35 Gew.-% - bezogen auf die Mittel - enthalten sein können.

### Beispiele

### Allgemeines Herstellbeispiel

In einem 1-1-Dreihalskolben mit Rührer, Innenthermometer und Destillationsaufsatz wurden 513 g (1,9 mol) teilgehärtete C_{16/18}-Talgfettsäure (lodzahl = 40), 1 mol Alkanolaminmischung und 1,4 g 50 Gew.-%ige unterphosphorige Säure gegeben. Über einen Zeitraum von 4 h wurde die Reaktionsmischung bei einem verminderten Druck von 40 mbar auf eine Temperatur von 160°C erhitzt, bis die Säurezahl unterhalb von 5 lag. Anschließend wurde der rohe Talgfettsäurealkanolaminester abgekühlt, der Reaktionsansatz entspannt und 0,4 g Wasserstoffperoxid zugegeben. Anschließend wurde in einem 500-ml-Dreihalskolben mit Rührer, Tropftrichter und Rückflußkühler eine Mischung von 45 g (0,072 mol) des Esters in 105 g Isopropylalkohol vorgelegt und unter Rühren auf 45°C erhitzt. Innerhalb von 2 h wurden 8,5 g (0,067 mol) Dimethylsulfat zugetropft. Nach Beendigung der Zugabe wurde die Mischung weitere 2 h bei 60°C gerührt. Die Mischung wurde als hellfarbiges Öl in praktisch quantitativer Ausbeute erhalten.

### Anwendungstechnische Beurteilung

Anschließend wurden die verschiedenen Esterquats mit Wasser auf eine Konzentration von 5 Gew.-% verdünnt und die Viskosität nach Brookfield sofort bzw. nach Lagerung (4 Wochen, 40°C) in einem RVT-Viskosimeter (20°C, Spindel 1, 10 Upm) bestimmt. Zur Untersuchung des Weichgriffs wurde Baumwollgewebe mit einem handelsüblichen Universalwaschmittel gewaschen, wobei man in den letzten Waschgang Esterquats unterschiedlicher Zusammensetzung hinzugab. Anschließend wurde der Griff der Gewebe von einem Panel aus 6 geschulten Personen beurteilt. Bezüglich des Weichgriffes bedeutet (1) sehr weich und 5 (hart). Die biologische Abbaubarkeit gemäß GF-Test wurde gegenüber einem Standard bestimmt. Die Ergebnisse sind in Tabelle 1 zusammengefaßt. Die Beispiele 1 und 2 sind erfindungsgemäß, die Beispiele V1 bis V4 dienen zum Vergleich.

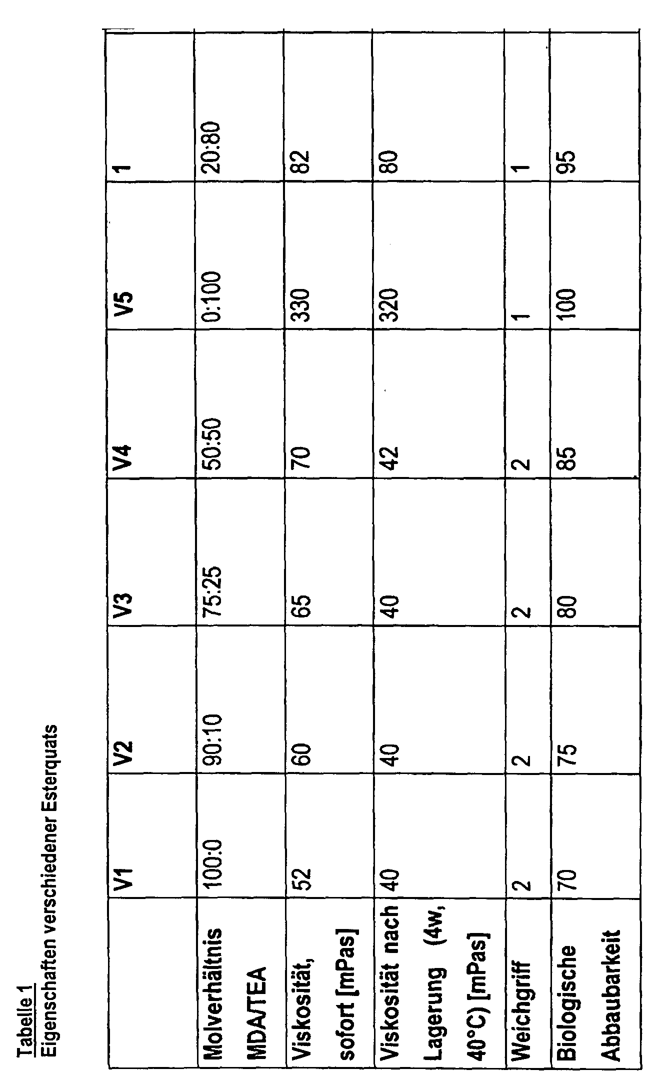

## Patentansprüche

1. Esterquats, dadurch erhältlich, dass man Mischungen von Methyldiethanolamin und Triethanolamin im Gewichtsverhältnis 20 : 80 bis 1 : 99 mit Fettsäuren der Formel (I)
**R**^{**1**}**COOH** **(I)**
in der R¹CO für einen aliphatischen, gesättigten und/oder ungesättigten Acylrest mit 6 bis 22 Kohlenstoffatomen steht, verestert und die Reaktionsprodukte anschließend in an sich bekannter Weise mit Alkylierungsmitteln quaterniert.

2. Verfahren zur Herstellung von Esterquats, **dadurch gekennzeichnet, dass** man Mischungen von Methyldiethanolamin und Triethanolamin im Gewichtsverhältnis 20 : 80 bis 1 : 99 mit Fettsäuren der Formel (I),
**R**^{**1**}**COOH** **(I)**
in der R¹CO für einen aliphatischen, gesättigten und/oder ungesättigten Acylrest mit 6 bis 22 Kohlenstoffatomen steht, verestert und die Reaktionsprodukte anschließend in an sich bekannter Weise mit Alkylierungsmitteln quaterniert.

3. Verfahren nach Anspruch 2, **dadurch gekennzeichnet, dass** man Fettsäuren mit 12 bis 18 Kohlenstoffatomen einsetzt, die eine lodzahl im Bereich von 0,5 bis 50 aufweisen.

4. Verfahren nach den Ansprüchen 2 bis 3, **dadurch gekennzeichnet, dass** man die Alkanolamine und die Fettsäuren im Molverhältnis 1 : 1,1 bis 1 : 2,4 einsetzt.

5. Verfahren nach den Ansprüchen 2 bis 4, **dadurch gekennzeichnet, dass** man die Alkylierung mit Dimethylsulfat durchführt.

6. Verwendung von Esterquats nach Anspruch 1 zur Herstellung von Avivagemitteln.

7. Verwendung von Esterquats nach Anspruch 1 zur Herstellung von kosmetischen Zubereitungen.

## Claims

1. Esterquats obtainable by esterifying mixtures of methyl diethanolamine and triethanolamine in a ratio by weight of 20:80 to 1:99 with fatty acids corresponding to formula (I):
R¹COOH (I)
in which R¹CO is an aliphatic, saturated and/or unsaturated acyl group containing 6 to 22 carbon atoms,
and then quaternizing the reaction products with alkylating agents by methods known per se.

2. A process for the production of esterquats, **characterized in that** mixtures of methyl diethanolamine and triethanolamine are esterified with fatty acids corresponding to formula (I):
R¹COOH (I)
in which R¹CO is an aliphatic, saturated and/or unsaturated acyl group containing 6 to 22 carbon atoms,
in a ratio by weight of 20:80 to 1:99 and the reaction products are then quaternized with alkylating agents by methods known per se.

3. A process as claimed in claim 2, **characterized in that** C₁₂₋₁₈ fatty acids with an iodine value of 0.5 to 50 are used.

4. A process as claimed in claims 2 and 3, **characterized in that** the alkanolamines and the fatty acids are used in a molar ratio of 1:1.1 to 1:2.4.

5. A process as claimed in claims 2 to 4, **characterized in that** the alkylation is carried out with dimethyl sulfate.

6. The use of the esterquats claimed in claim 1 for the production of softeners.

7. The use of the esterquats claimed in claim 1 for the production of cosmetic preparations.

## Revendications

1. Esterquats,
**caractérisé en ce qu'**
ils sont accessibles en estérifiant des mélanges de méthyldiéthanolamine et ditriéthanolamine dans un rapport pondéral allant de 20 : 80 à 1 : 99, avec des acides gras de formule (I) :
R¹COOH (I)
dans laquelle R¹CO représente un reste acyle aliphatique saturé et/ou non saturé ayant de 6 à 22 atomes de carbone,
et en quaternisant ensuite les produits de la réaction d'une manière connue en soi avec des agents d'alkylation.

2. Procédé de production d'esterquats,
**caractérisé en ce qu'**
on estérifie des mélanges de méthyldiéthanolamine et de triéthanolamine dans un rapport pondéral de 20 : 80 à 1 : 99 avec des acides gras de formule (I) :
R¹COOH (I)
dans laquelle R¹CO représente un reste acyle aliphatique saturé et/ou non saturé ayant de 6 à 22 atomes de carbone et ensuite on quaternise les produits de la réaction d'une manière connue en soi, avec des agents d'alkylation.

3. Procédé selon la revendication 2,
**caractérisé en ce qu'**
on met en oeuvre des acides gras ayant de 12 à 18 atomes de carbone, qui possèdent en indice d'iode dans la zone de 0,5 à 50.

4. Procédé selon les revendications 2 et 3,
**caractérisé en ce qu'**
on met en oeuvre les alkanolamines et les acides gras dans un rapport molaire de 1 : 1,1 et 1 : 2,4.

5. Procédé selon l'une quelconque des revendications 2 à 4,
**caractérisé en ce qu'**
on effectue l'alkylation avec du sulfate diméthylique.

6. Utilisation des esterquats selon la revendication 1 en vue de la préparation d'agents d'avivage.

7. Utilisation des esterquats selon la revendication 1 en vue de la production de préparation cosmétiques.
